Europäisches Patentamt

(19) European Patent Office

Office européen des brevets

(11) Publication number : **0 512 011 B1**

(12) **EUROPEAN PATENT SPECIFICATION**

(45) Date of publication of patent specification :
06.04.94 Bulletin 94/14

(51) Int. Cl.⁵ : **C12N 15/57,** C12N 15/12,
C12N 15/86, C12N 5/10,
C12N 15/54

(21) Application number : 91903050.2

(22) Date of filing : 24.01.91

(86) International application number :
PCT/EP91/00139

(87) International publication number :
WO 91/11519 08.08.91 Gazette 91/18

(54) RECOMBINANTLY PRODUCED BLOOD FACTORS AND PROCESS FOR THE EXPRESSION OF SAID BLOOD FACTORS, AS WELL AS VACCINIA VIRUS RECOMBINANTS USED IN SAID PROCESS.

(30) Priority : 26.01.90 EP 90101623

(43) Date of publication of application :
11.11.92 Bulletin 92/46

(45) Publication of the grant of the patent :
06.04.94 Bulletin 94/14

(84) Designated Contracting States :
AT BE CH DE DK ES FR GB GR IT LI LU NL SE

(56) References cited :
EP-A- 0 198 328
EP-A- 0 303 540
EP-A- 0 338 807
EP-A- 0 373 012
WO-A-88/03926
WO-A-89/12685

(73) Proprietor : IMMUNO Aktiengesellschaft
Industriestrasse 67
A-1221 Wien (AT)

(72) Inventor : FALKNER, Falko-Günter
A-2304 Mannsdorf 116 (AT)
Inventor : MACGILLIVRAY, Ross, T., A.
Apt. 807-2233 Allison Road
Vancouver, British Columbia V6T 1W5 (CA)
Inventor : BODEMER, Walter
Hessegasse 32/31
A-1228 Wien (AT)
Inventor : SCHEIFLINGER, Friedrich
Raiffeisenstrasse 15
A-2304 Orth/Donau (AT)
Inventor : EIBL, Johann
Gustav Tschermakgasse 2
A-1180 Wien (AT)
Inventor : DORNER, Friedrich
Peterlinigasse 17
A-1230 Wien (AT)

(74) Representative : Kolb, Helga, Dr. Dipl.-Chem.
et al
Hoffmann, Eitle & Partner, Patentanwälte,
Postfach 81 04 20
D-81904 München (DE)

EP 0 512 011 B1

**Description**

Recombinantly produced blood factors and process for the expression of said blood factors, as well as vaccinia virus recombinants used in said process

The present invention concerns recombinantly produced blood factors, especially vitamin K-dependent blood clotting factors, vaccinia virus recombinants, and a process for the expression of said blood factors. The invention discloses especially the preparation of recombinant prothrombin and thrombin as well as intermediates which are formed by differing processing mechanisms on the pathway from prothrombin to various variants of thrombin.

There is a demand for highly purified blood factors such as Factor VIII, Factor II, von Willebrand Factor and plasminogen which are not contaminated by any infectious or toxic material. Genetic engineering offers a way to produce such prophylactic or therapeutically significant proteins in comparatively high yields and in the absence of pathogenic viruses such as HIV and hepatitis viruses. Eukaryotic expression systems provide the further advantage to carry out post-translational modifications at the protein moiety so that glycoproteins are obtained which are highly comparable or identical to the substances produced by the human body.

Several vitamin K-dependent human clotting factors have been expressed in various recombinant systems. The expression of human clotting factor IX has been reported in hepatoma cells and in murine fibroblasts by H. de la Salle et al in Nature 316, 268-270 (1985) and D.S. Anson et al in Nature 315, 683-865 (1985), in baby hamster kidney cells by S. Busby et al in Nature 316, 271-273 (1985) and in Chinese hamster ovary (CHO) cells by N.J. Jorgensen et al in Cell 48, 185-191 (1987). From these studies it is known that only certain cell types, such as liver and kidney derived cell lines, possess efficient carboxylation systems. Further, it has been observed that overexpression of Factor IX in CHO cells by gene amplification usually results in elevated levels of protein antigen, however, the specific activity drops drastically and also processing from the prepro-protein into the mature form is insufficient (A. Balland et al in Eur. J. Biochem. 172, 565-572 [1988]).

Another vitamin K-dependent factor, human protein C, has been expressed in a human 293 embryonal kidney cell system. In this system high level expression of fully carboxylated protein C was achieved (J.D. Walls et al in Gene 81, 139-149 [1989]). The 293 cell line, however, does not seem to be suitable for scale-up and fermentation.

Recombinant studies have also been carried out with prothrombin. The vitamin K-dependent coagulation factor prothrombin is a plasma glycoprotein with a molecular weight of 72,000. This protein which is synthesized in the liver is involved in the final stages of blood coagulation. Prior to secretion it undergoes several post-translational modifications, such as glycosylation, vitamin K-dependent carboxylation of the first 10 amino-terminal glutamic acid residues and cleavage of the pre- and propeptides. The cloning of the gene for human prothrombin and its complementary DNA (S.J.F. Degen et al, Biochemistry 26, 6165-6177 [1987]) has opened up the possibility for expression studies in appropriate cell systems. As to the full-length cDNA of prothrombin including the signal peptide region and pro-sequence (coding for prepro-prothrombin) reference is made to MacGillivray, R.T.A., Irwin, D.M. Guinto, R.E. and Stone, J.C. 1987, "Recombinant genetic approaches to functional mapping of thrombin", Annals of the New York Academy of Sciences 485, 73-79. Expression of active human prothrombin has been achieved in Chinese hamster ovary (CHO) cells and in baby hamster (BHK) kidney cells by M.J. Jorgensen et al, J. Biol. Chem. 262, 6729-6734 (1987) . When the expression of prothrombin in CHO cells was enhanced by gene amplification, high expression levels were obtained, however, only 60 % of the material was sufficiently carboxylated. The expression of prothrombin in BHK cells yielded active prothrombin, however, the expression levels were comparatively low.

Vaccinia virus based vector systems are known e.g. from M. Mackett et al, in D.M. Glover (ed), DNA cloning: A practical approach, IRL Press, Oxford, p. 191-211 (1985). In EP-243,029 a recombinant vaccinia virus is described for the expression of the HIV env protein.

Vaccinia virus has evolved its own transcriptional regulatory sequences which are recognized by a virus-encoded RNA polymerase packaged into the infectious virus particle. In addition, vaccinia virus has a large genome (185 kilo base pairs of double stranded DNA), which cannot be handled by in vitro cloning techniques. Insertion of foreign DNA into the vaccinia virus genome can therefore be accomplished only in vivo by exploiting the principle of general homologous recombination.

Vaccinia virus has proved to be a useful means for gene expression studies in mammalian cells. Advantages include the maintenance of infectivity, wide host range, large DNA capacity and correct synthesis, folding, processing and transport of proteins.

Recombinant vaccinia virus therefore provides a rapid means for screening the cell lines that are able to carry out the correct post-translational modifications and are - in combination with an appropriate host cell - also efficiently secreting the desired protein. This is especially important when the expression of extensively

2

modified secretory proteins, such as the vitamin K-dependent coagulation factors, is considered.

The object of the present invention is to provide an expression system for blood factors, especially vitamin K-dependent blood clotting factors, such as prothrombin and thrombin variants, which is improved over the expression systems so far known in that it offers a high post-translational capacity and, due to the stable viral recombinants, very reproducible expression conditions.

A further object is to provide recombinantly prepared blood factors or derivatives thereof which have a high ratio of the biologically active form to the antigenic form.

The above object is solved by recombinantly produced blood factors which have a ratio of their biologically active form to the antigenic form of at least 50 %, preferably in the range of 60 to 90%, and most preferably in the range of 70 to 80 %.

Among these blood factors, which may comprise the entire or partial amino acid sequence of the protein as well as derivatives therefrom by replacement, deletion or insertion of at least one amino acid, Factor V, Factor VIII, Factor XII, Factor XIII, von Willebrand Factor, plasminogen, and vitamin H-dependent blood factors, such as Factor VII, Factor IX, Factor X, protein C, protein S and especially prothrombin as well as variants thereof are mentioned.

The invention also comprises insertion plasmids and vaccinia virus recombinants necessary for the expression of the desired glycoproteins.

It is further suggested to use other recombinant pox viruses, e.g. fowlpox virus.

The insertion plasmids comprise a foreign cDNA sequence coding for the desired blood factor and at least one replicon, at least one selection marker, multiple cloning sites, at least one promoter, and DNA sequences from vaccinia virus, which flank the promoter and the foreign DNA sequence.

The selection of virus recombinants or cells containing the same is facilitated by the use of a selection marker. The expression of this marker is preferably under the control of a strong, continuously active promoter, for instance P 7.5 from vaccinia virus. An example for a suitable selection marker is the gpt gene which codes for the xanthine guanine phosphoribosyl transferase. Only a recombinant virus which carries the gpt gene will grow in a mammalian cell culture in the presence of mycophenolic acid (F.G. Falkner and B. Moss in J. Virol. 62, 1849-1854 [1988]).

While it is of utmost importance that the foreign gene to be expressed is under the control of a poxvirus promoter, it is equally important that this poxvirus promoter, e.g. a vaccinia promoter, is in immediate proximity to the foreign gene sequence to be expressed. It has been shown in numerous examples that untranslated coding regions which are situated between the poxvirus promoter and the foreign gene sequence to be expressed result in a reduction of the expression level. Special care should therefore be given that the 3'-terminal end of the promoter and the 5' foreign gene sequence following down-stream thereof should be aligned in proper reading frame, thereby avoiding unnecessary regions of untranslated codons. One possibility to achieve this is to choose an appropriate restriction site and to modify the 5'-end of the foreign gene sequence (possibly including any pre- or prepro-sequence) by adding or deleting codons in a way that an identical restriction site is formed. In this way oberlapping ends of the 3'-end of the promoter and the 5'-end of the foreign gene allow the desired assembly.

The choice of the appropriate promoter in a vaccinia virus expression system is of great importance. Usually, viral promoters active in the late phase or active in the early and late phase of the' infection cycle are used for protein expression.

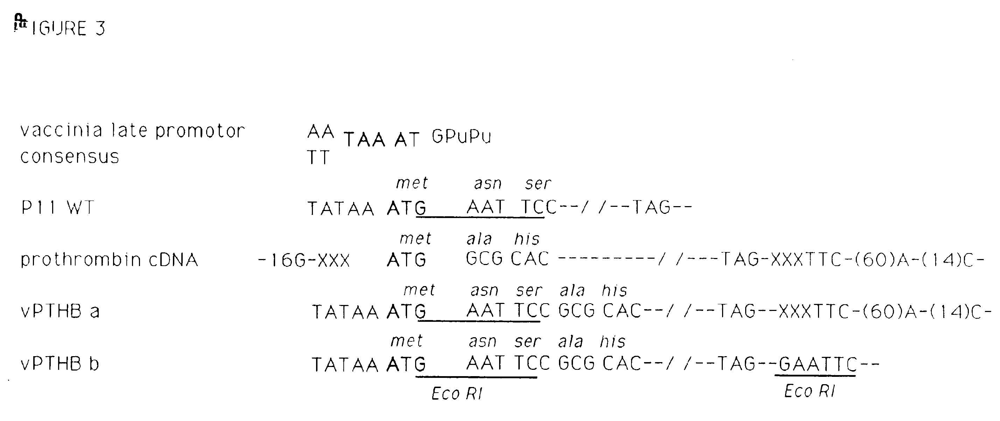

According to the invention vaccinia virus late 11 K-promoter is used. This promoter contains a functionally important conserved sequence element that overlaps with the transcriptional and the translational initiation sites (see B. Moss et al, Ann. Rev. Immunol. 5, 305-324 [1987]). In order to get efficient expression, the inventors have chosen not to alter this region. Therefore, in the example given in the following the prothrombin cDNA has been cloned into the naturally occurring EcoRI site immediately downstream of the initiation codon of the 11 K polypeptide, as shown in Fig.3.

As described in more detail in the experimental section, the exact fusion of the promoter with the prothrombin coding region was obtained by introduction of a novel EcoRI site by means of oligonucleotide directed mutagenesis downstream the initiation codon of the prothrombin gene. The strategy chosen resulted in the introduction of two additional amino acids (Asn and Ser) into the signal peptide (see Fig. 3). Since the signal peptide cleavage site is located about 40 amino acids away, this mutation does not interfere with the correct processing and secretion of the nature polypeptide chain.

Due to the fact that vaccinia virus DNA is transcribed in the cytoplasm, no use will be made of the host cell's splicing apparatus. Therefore, in constructing a virus recombinant, intron-free DNA or cDNA has to be used. For efficient expression and secretion it is preferable that this DNA comprises also signal sequences.

For some purposes it may be desirable to insert into the recombinant virus only a partial sequence or an amended sequence of the foreign DNA sequence to be expressed. In this way derivatives or mutants or activated forms of the blood factors are obtained.

The insertion plasmids further comprise flanking DNA sequences of vaccinia virus, which may correspond to the thymidine kinase gene or to hemagglutinine sequences, or to any non-essential region in the vaccinia virus DNA.

A suitable open reading frame vector for the insertion of the foreing cDNA sequence is pTKgpt-oF1s as described by F.G. Falkner and B. Moss in J. Virol. 1988, 62, 1849-1854.

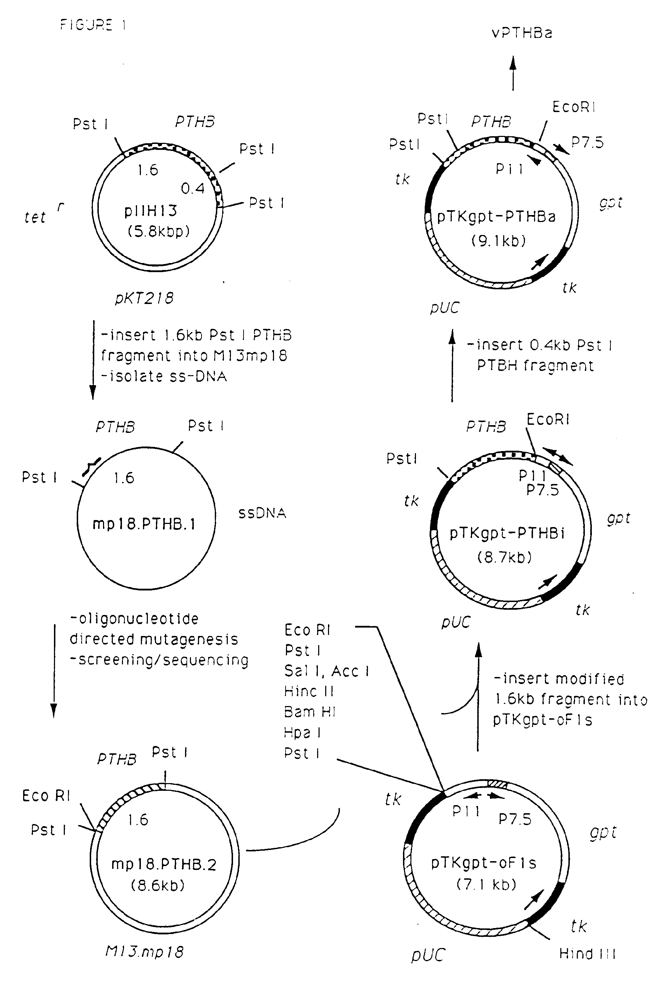

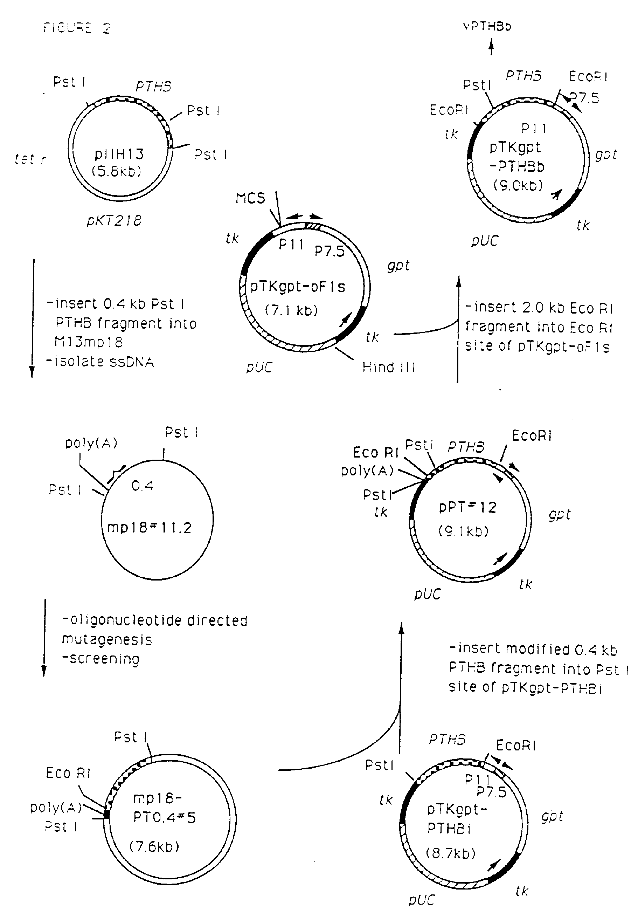

With the help of this open reading frame vector, the insertion plasmid pTKgpt-PTHBa and pTKgpt-PTHBb (Fig.1 and 2) have been prepared. In a similar way as described for prothrombin any other desirable foreign gene coding for a blood factor may be inserted into plasmid pTKgpt-oF1s in close proximity ot the P11 promoter.

With such insertion plasmids the vaccinia virus recombinants are obtained by in vivo homologous recombination with wild-type vaccinia virus. The vaccinia virus recombinants described in the experimental section for the expression of prothrombin are vPTHBa and vPTHBb (Fig. 1 and 2) as well as vPT6/2, vTKemc-PT2, vHA-PTa und vHA-PTb which will be described below.

With these seed viruses a mammalian cell culture is then infected and screened for the expression of the desired glycoprotein.

As mammalian host cells, liver and kidney cells are preferred since they offer the most sufficient post-translational modification. It could be shown that most preferable as host cells are Vero, CV1, BSC1 kidney cells as well as BHK cells in view of expression levels and post-translational modification.

Other useful host cells are RK13 cells. These cells do not secrete the desired protein into the supernatant, but they have the ability to express the protein in huge amounts. In this case, the cells have to be destroyed in order to recover the protein. Thus, in this case it may be advantageous to express the desired protein without any signal sequence.

The process for the production of the desired recombinant blood factors is preferably carried out in the presence of vitamin K in order to obtain proteins which possess high biological activity.

A further improvement of the expression system is achieved by observing the phase of the cell when infection is carried out. It has proved that best results are obtained when the cells have reached confluency prior to infection.

As to the amount of vaccinia virus recombinants necessary for infection of the host cells, it could be determined that the same lies preferably in the range of 0.1 to 20 pfu, most preferably in the range of 0.1 to 1 pfu. Higher amounts of virus recombinants used for infection did not result in more elevated expression levels.

Expression levels have further been increased by employing high density systems, such as micro-carrier cultures or hollow fiber systems.

Proteinaceous material thus produced can be harvested from the cell supernatant or the cells by any suitable technique known in the art, and purified by employing common methods of protein chemistry.

The vaccinia virus recombinants possess a significant advantage over the prior art recombinant systems in that they allow the rapid screening of cell lines for efficient secretion and correct post-translational modifications. In this way it can be easily established which cell lines are able to secrete large amounts of active protein, while others may accumulate large amounts in their cytoplasm, and which cell lines yield the most active proteins due to their correct post-translational modification. It is therefore useful, before constructing a transformed cell line, to screen different cell types for the required functions by infection studies with vaccinia virus recombinants.

With the recombinantly produced human prothrombin, which is free from blood borne viruses such as e.g. HIV or NANB hepatitis viruses it is possible to treat certain disorders in the coagulation system with human thrombin, prepared from prothrombin, such as e.g. septic shock. Human thrombin and/or a synergistic mixture of human thrombin-thrombomodulin and protein C are further considered to be a useful drug for anticoagulant therapy and septic shock.

A further application is the use of thrombin produced from human recombinant prothrombin in a fibrin sealant instead of the currently used bovine thrombin. This fibrin sealant is extensively used in certain forms of surgery. Replacement of bovine by human thrombin can reduce the risk of certain virus infections as well as of allergic reactions.

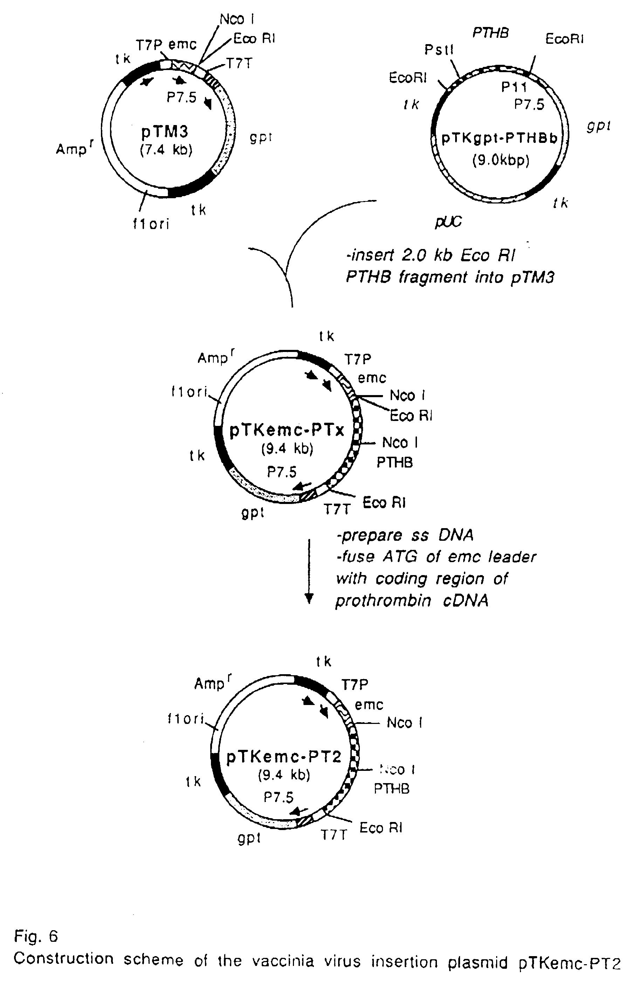

The invention is further illustrated by Fig. 1 to 7.

Fig. 1    shows the construction of vaccinia virus insertion vector pTKgpt-PTHBa. This insertion plasmid was used in an in vivo recombination event to construct the recombinant virus vPTHBa. The meaning of the abbreviations is as follows:

PTHB = prothrombin sequences; tetr = tetracycline resistence gene; ssDNA = single strand DNA; tk = vaccinia virus thymidine kinase sequences; gpt = E. coli xanthine guanine phosphoribosyl transferase gene; P11 = promoter of the vaccinia virus major late 11K polypeptide; P7.5 = promoter of the vaccinia virus 7.5 kDa polypeptide; MCS = multiple cloning sites. The arrows indicate the directions of transcription.

Fig. 2    shows the construction of vaccinia virus insertion vector pTKgpt-PTHBb. This insertion plasmid was used to construct the vaccinia virus recombinant vPTHBb. The abbreviations are as explained above for Fig. 1.

Fig. 3    shows the fusion of the prothrombin coding region with the vaccinia virus late P11-promotor (P11-WT). In the vaccinia virus recombinants vpTHBa and vPTHBb the coding region of human prothrombin is fused exactly behind the translational initiation codon of the 11 K polypeptide. Due to the insertion of a novel Eco RI restriction endonuclease cleavage site by oligonucleotide directed mutagenesis, the prothrombin signal peptide contains two additional amino acids (Asn and Ser). In the recombinant vPTHBb also the poly(A)-tail (60 A-residues present in the cDNA and in the recombinant vPTHBa) and an oligo(C)-stretch of 14 C-residues were removed by insertion of a second Eco RI site and subsequent cleavage.

Fig. 4    shows the construction scheme of vaccinia virus insertion plasmids pHAgpt-oFa and pHAgpt-oFb. HA = hemagglutinin; further abbreviations may be derived from previous Figures. For restriction endonucleases common abbreviations have been used. For further explanations see also Example III.

Fig. 5    shows the construction scheme of insertion plasmids pHAgpt-PTa and pHAgpt-PTb. For abbreviations see previous Figures.

Fig. 6    shows the construction scheme of vaccinia virus insertion plasmids pTKemc-PT2. Amp $^r$ = ampicilline resistence gene; emc = leader sequence of the encephalomyocarditis virus sequence; T7P = T7 polymerase sequence. For further abbreviations see previous Figures and Example IV..

Fig. 7    gives a comparison of expression levels induced by the different viral recombinants expressing prothrombin in Vero cells. For the experiment host cells were infected with 1 pfu per cell with the respective viral recombinant (in case of the T7 based vectors with 1 pfu each of the T7 polymerase providing virus and the T7 promoter target gene virus) and grown in serum-free medium in the presence of Vitamin $K_1$. Supernatants were harvested after 72 h and biological activities were determined as described in Example I.

In the following, experiments for the preparation of vaccinia virus insertion plasmids, virus recombinants, the expression of blood clotting factor prothrombin as well as experiments for the optimization of this expression are described. The following experiments serve to further illustrate the invention without restricting the same.

Example I:

Preparation of VV recombinants vPTHBa and vPTHBb and expression of prothrombin

1. Construction of vaccinia virus insertion plasmids.

a) Contruction of pTKgpt-PTHBa

The 1.6 kb Pst I subfragment of the prothrombin cDNA (excised from plasmid pIIH13, as described by R.T.A. MacGillivray, D.M. Irwin, R.E. Guinto and J.C. Stone in "Recombinant genetic approaches to functional mapping of thrombin", Annals of the New York Academy of Sciences 485, 73-79 (1987)) was cloned into the single strand phage M13mp18 and an Eco RI site was introduced by oligonucleotide directed mutagenesis using a phosphorothionate-based mutagenesis procedure (Amersham, Inc.). The oligonucleotide oPT1 (5'-TCG GAC GTG CGC GGA ATT CAT AGT GTG TCA-3') was used for the mutagenesis (the Eco RI site is underlined). The oligonucleotide oPT2 (5'-CTG TGC ACA AGG CTA CAC-3') is located about 60 bp downstream and serves as the sequencing primer to control the mutation. The novel Eco RI-Pst I fragment was then cloned into the open reading frame expression vector pTKgpt-oF1s, which has been described by F.G. Falkner and B. Moss in J. Virol. 62, 1849-1854 (1988). To complete the prothrombin coding region, the 0.4 kb Pst I subfragment of prothrombin cDNA was cloned into the single Pst I site of the intermediate plasmid pTKgpt-PTHBI resulting in plasmid pTKgpt-PTHBa. The construction of this insertion plasmid is schematically represented in Fig. 1.

b) Construction of pTKgpt-PTHBb

In this case the 0.4 kb Pst I subfragment of the prothrombin cDNA (excised from pIIH13 and comprising the signal- and pro-sequences) was cloned into M13mp18 and an Eco RI site was introduced by oligonucleotide directed mutagenesis 70 bp downstream of the stop codon. The oligonucleotide oPT3 (5'-GTT TCT AAA ACT AGA ATT CCC AAT AAA AGT-3') was used for mutagenesis, the oligonucleotide oPT5 (5'-ATT CTG GGC TCC TGG A-3'), located about 60 bp downstream of the mutation, served as the sequencing primer to control the mutation. The modified 0.4 kb Pst I fragment was cloned into the single Pst I site of the intermediate plasmid pTKgpt-PTHBI (see construction of pTKgpt-PTHBa as described above). The 2.0 kb Eco RI fragment containing prothrombin cDNA without the poly(A) sequence was cloned into the single Eco RI site of pTKgpt-oF1s resulting in plasmid pTKgpt-PTHBb. The construction of this plasmid is schematically outlined in Fig. 2.

In both of the above described plasmids, the prothrombin coding region is exactly fused behind the initiation codon of the 11 K polypeptide. The scheme to obtain this exact fusion is represented in Fig. 3. As can be derived from Fig. 3, the strategy chosen for the introduction of a novel Eco RI site by oligonucleotide directed mutagenesis resulted in the incorporation of two additional amino acids (Asn and Ser) into the signal peptide of prothrombin. Since the signal peptide cleavage site is located about 40 amino acids away, this mutation was likely not to interfere with the correct processing and secretion of the mature polypeptide chain.

It can further be derived from Fig. 3 that in pTKgpt-PTHBb, in addition, the poly(A)-tail of the prothrombin cDNA and a 14 bp G/C sequence has been removed by introducing a second Eco RI site at the 3'-end of the prothrombin gene and the subsequent cleavage, while in pTKgpt-PTHBa the poly(A) tail and the 14 bp G/C sequence are still present.

## 2. Construction of vaccinia virus recombinants vPTHBa and vPTHBb

For in vivo recombination the procedure described by M. Macket et al in "The construction and characterization of vaccinia virus recombinants expressing foreign genes", D.M. Glover (ed.), DNA cloning: A practical approach, IRL Press, Oxford (1985), p. 191-211, was carried out with the following modifications:

$5 \times 10^6$ CV1 cells (confluent monolayers) were infected with 0.2 pfu/cell of vaccinia wild-type virus. Two hours after infection 1 ml of a calcium-DNA-precipitate (consisting of 5µg of supercoiled plasmid DNA of pTKgpt-PTHBa or pTKgpt-PTHBb, respectively, 1µg of vaccinia virus wild-type DNA and 14 µg of sheared herring sperm DNA) was added to the cells. After 15 minutes of incubation at room temperature 9 ml of medium (DMEM, 8 % FBS with antibiotics) were added. The medium was changed after 4 h and the incubation was continued for another 48 h. Cells were harvested and resuspended in 1 ml of medium and a viral crude stock was prepared.

## 3. Selection of gpt[+] vaccinia virus recombinants

For the isolation of gpt[+] mutants, a plaque assay on BSC1 cells was done as follows: confluent BSC1 cells were preincubated in the gpt selective medium (DMEM, 2,5 % FBS, antibiotics, 25 µg/ml MPA, 250 µg/ml xanthine and 15 µg/ml hypoxanthine) for 14-24 h. The $10^{-3}$, $10^{-4}$ and $10^{-5}$ dilutions of a viral crude stock (0,5 ml of the resuspended cells were frozen and thawed 3 times, an equal volume of 0.25 mg/ml trypsin was added, the mixture was digested for 30 min at +37°C and sonicated for 20 seconds) were used to infect the BSC1 cells. After 1.5 h of incubation at +37°C the cells were overlaid with the gpt-selective medium containing 1 % of low melting agarose. After 2 days of incubation the cells were stained with neutral red. The plaques were readily visible after overnight incubation. The plaque purifications were carried out as described by F.G. Falkner et al in J. Virol. 62, 1849-1854, 1988.

## 4. Screening for prothrombin expressing recombinant virus

### a) Infection of cells with seed virus

Monkey kidney cells (CV1 and Vero cells) were grown in DMEM, 10 % fetal calf serum with antibiotics and glutamine. One day before reaching confluency, vitamin $K_1$ was added in the final concentration of 20 µg/ml (vitamin $K_1$ was obtained from Sigma, #V-3501, stored at +4°C as a 5 mg/ml stock solution in ethanol). When the monolayers reached confluency, they were washed twice in phosphate buffered saline containing $Mg^{2+}$ and $Ca^{2+}$ and were subsequently infected with 0.1 to 10 plaque forming units (pfu) of purified virus. After 1 h of virus adsorption, serum-free medium (DMEM) and vitamin K were added.

The cells were grown for 2 days and subsequently the proteins of the cellular supernatants were precipitated and analyzed by Western blotting with an alkaline-phosphatase coupled antibody against human prothrombin as described in the following:

b) Acetone precipitation of cell culture supernatants

The supernatants were centrifuged for 10 min at 3000 g to remove cell debris, precipitated with two volumes of acetone and incubated for 30 min on ice. After centrifugation for 10 min at 8000 g, the pellet was redissolved in 0.25 ml of 0.01 N NaOH, heated for 3 min at 70°C and centrifuged to remove undissolved material. The supernatant was neutralized with 20 µl of 1M Tris/HCl pH 7.0 and again precipitated with 2 volumes of acetone. The resulting pellet was dissolved in 40 µl of SDS-sample buffer. Usually 10 µl were loaded onto the gel.

c) Western blot analysis

The procedure was essentially carried out as described by H. Towbin et al in Proc. Natl. Acad. Sci. USA 83, 6672-6676 (1979) with the following modifications: The proteins were separated on a 10% SDS-polyacrylamide gel and were blotted onto a nitrocellulose membrane (Amersham Hybond C) for 1 hour at 200mA in a Hoefer TE series Transphor Electrophoresis unit in a transfer buffer containing 12.5 mM Tris, 96 mM glycine pH 8.3 and 10 % methanol. The membrane was soaked 3 x 20 min in Western buffer (WB, phosphate buffered saline, 0.1 % Tween 20) and preincubated in 20 ml WB containing 10 % normal sheep serum. After 1 hour 40 µl of a sheep anti-human prothrombin antibody coupled with alkaline phosphatase (Serotec AHP 06A) was added (resulting an a 1:500 dilution of the antiserum) and the incubation was continued overnight. The membrane was washed 3 x 10 min in WB, 1 x 3 in alkaline phosphatase (AP) buffer (0.1 M Tris/CHl pH 9.5, 0.1 M NaCl, 5 mM MgCl$_2$) and subsequently developed in 10 ml AP buffer containing 66 µl nitroblue tetrazolium (NBT; 50 µg/ml in 70 % dimethylformamide) and 33 µl 5-bromo-4-chloro-3-indolyl phosphate (BCiP; 50 µg/ml in dimethylformamide) for 3 to 10 min.

In all samples a novel protein band was readily visible co-migrating with the purified prothrombin control, while this band was absent in non-infected or wild-type infected cells. Based on this screening step, positive viruses vPTHBa and vPTHBb were grown to large scale and purified.

d) Genomic characterization of the viral recombinants

In order to check for the presence and the integration pattern of the prothrombin gene in the recombinant virus a restriction enzyme cleavage followed by a Southern blot analysis was performed. DNA of the purified recombinants vPTHBa and vPTHBb was digested with the restriction endonucleases Hind III and Eco RI, electrophoresed through a 1 % agarose gel and blotted onto a nitrocellulose filter. The filter was first hybridized with a thymidine kinase probe (tk-probe) and subsequently with a prothrombin gene probe (pTHB-probe):

In the Hind III digested samples, hybridized with the tk-probe, the wild-type virus Hind III J-fragment that contained the thymidine kinase gene was visible as a band of about 5.0 kb. Both recombinant viruses contained the anticipated band of about 6.0 kb and the smaller one of about 1.0 kb that contained tk-sequences. With the prothrombin-probe only the 6.0 kb band was visible. The patterns obtained confirm the integration of the prothrombin cDNA into the viral tk-locus.

In the Eco RI digested samples, hybridized with the tk-probe, the thymidine kinase gene of the wild-type virus was split into two fragments of about 7.0 and 8.0 kb. In the vPTHBa sample, due to the integration of the prothrombin gene, the two anticipated larger fragments of about 9.0 and 10.0 kb appeared. In vPTHBb a second Eco RI site was introduced that lead to the excision of the prothrombin gene upon Eco RI digestion. The vPTHBb pattern therefore showed the 10.0 kb fragment (also seen in the vPTHBa sample) and the 7.0 kb fragment (also seen in the wild-type DNA). With the prothrombin probe in vPTHBa the expected 9.0 kb fragment and in the case of vPTHBb the 2.0 kb excised prothrombin gene was detected. The analysis confirmed the expected integration pattern of the two recombinant viruses.

5. Selection for preferred cell lines secreting prothrombin

The potential of cells for certain functions such as e.g. growth, adhesion on substrates, secretion and post-tranlational modification varies from cell type to cell type. The following experiment serves to screen and identify cell lines that lie within the vaccinia virus host range, have acceptable growth characteristics and are able to secrete active vitamin K dependent proteins.

Prothrombin activity was determined in a clotting assay using prothrombin deficient plasma. Human prothrombin of known concentration (Immuno FII, IX, X-CPK standard) was used to prepare a standard curve.

For the experiments shown in Table 1 the cells were infected with vPTHBa. The supernatants and the total cellular proteins, harvested after 48 h, were analyzed by SDS-polyacrylamide gel electrophoresis and quantified relative to known amounts of purified prothrombin in a Coomassie blue stained gel.

Besides liver cell lines (Hep G2 and H4-11-E-C3) also kidney cells (e.g. BHK and MDCK) and ovary cells (CHO) have been shown to produce active gamma-carboxylated proteins. Based on the known properties of these cell lines and the requirements for the expression of biologically active prothrombin, the cell lines as outlined in Table 1 have been chosen for prothrombin expression studies.

T a b l e    1

Selection of a prothrombin secreting cell line

| Cell line | Source | 48 h sup. | intracell. | carboxylation |
|---|---|---|---|---|
| BSC1 | kidney, AGM (CCl 26) | +++ | + | n.d. |
| CV1 | kidney, AGM (CCL 70) | +++ | ++ | yes |
| Vero | kidney, AGM (CCL 81) | +++ | + | yes |
| RK 13 | kidney, rabbit (CCL 37) | + | +++ | n.d. |
| 143B | osteosarcoma (CRL 8303) | – | + | n.d. |
| 293 | kidney, human (CRL 1573) | + | + | yes |
| BHK-21 | kidney, hamster (CCL 10) | + | ++ | yes |

+   =   visible prothrombin band (ca. 50 ng)

++  =   well visible (ca. 500 to 100 ng)

+++ =   strong band (> 200 ng)

–   =   no prothrombin band

AGM =   African green monkey

n.d.=   not determined

From Table 1 it can be seen that the African green monkey kidney cell lines BSC1, CV1 and Vero cells most sufficiently secrete prothrombin. For the rabbit kidney cell line RK13 it could be shown that the same accumulates large amounts of the protein intracellularly. Secretion and accumulation of prothrombin was also found in kidney cell lines 293 and BHK-21 when infected by the recombinant virus according to the invention.

The vaccinia virus recombinants according to the invention allow the rapid screening of cell lines for the expression of blood factors, especially the vitamin K-dependent blood clotting factors. The migration in the same size range suggests that the recombinant prothrombin derived from the vaccinia virus infected cells have a similar degree of glycosylation as the blood derived protein.

6. Optimization of the expression of biologically active prothrombin

Since the above experiment has shown that the prothrombin levels in the supernatants of infected CV1 and Vero cells were significantly higher than in the other cell lines tested and the preliminary coagulation assays had shown that the supernatants were biologically highly active, these two cell lines were selected for further experiments.

a) In the first experiment the dependence of the activity of the cell culture supernatants from the dose of infection with the prothrombin expressing virus was determined. It could be shown that in the range between 0.1 and 20 plaque forming units per cell of vPTHBa or vPTHBb the prothrombin levels in the 48 h supernatants did not differ significantly. One may therefore derive that 0.1 to 3, or preferably 0.1 ot 1 plaque forming unit per cell is sufficient to obtain the maximal amount of secreted factor II in both, CV1 and Vero cells.

b) The following experiment concerns the accumulation of the secreted protein with increasing incubation time. Cell culture supernatants of infected CV1 cells were examined after 12, 24, 48, 60, 72 and 96 hours post infection. A continuous increase in secreted prothrombin was observed over the time range up to 72 hours. These tests have been accompanied by gel electrophoresis and Western blot analysis. It was observed in the gels that over a prolonged time there is also an increase in other secreted proteins. A maximum of secreted prothrombin was shown to be between 60 and 72 hours.

c) Cell cycle dependence of prothrombin expression

Another essential parameter for efficient expression in the system according to the invention turned out to be the phase of the cell cycle of the infected cells. For this experiment CV1 cells were grown in the presence of vitamin $K_1$ until they just reached confluency, or they were grown for another one, two or three days prior to infection. The supernatants were examined for factor II activity at 48 and 72 hours post infection.

It was observed that when infection was carried out immediately after the cells had reached confluency (1 day) moderate activity levels were obtained. However, with increasing age of the confluent monolayer, a continuous increase of prothrombin was observed. The maximum of prothrombin activity was obtained after infecting CV1 cell that had reached confluency. It has been shown in repeated experiments that arresting the cells prior to infection in a stationary phase yielded best results. The elevated expression levels are partly due to the increasing cell density, but increased density alone does not fully account to overall expression increase.

7. Calculation of expression levels

The expression levels as obtained in the above described experiments in CV1 and BSC1 cells were determined to yield up to 8.0 µg/ml of prothrombin antigen.

8. Determination of biological activity

The biological activity of the recombinant prothrombin was determined to lie in the range of 50 - 70 milliunits per ml of culture medium, thus yielding a recombinant prothrombin with a ratio of biologically active form to antigenic form in Vero cells of up to 70 %
in CV1 cells of up to 60 %.

Example II:

Construction of the vaccinia virus recombinant vPT6/2 and expression of prothrombin

To construct the recombinant virus vPT6/2 the insertion plasmid pTKgpt-PTHBb as shown in Fig. 2 is recombined with vaccinia virus strain WR6/2 (Moss et al: Deletion of a 9,000 bp segment of the vaccinia virus genome that encodes non-essential polypeptides; J. Virol. 40, 1981, 387-395; this VV strain is publicly available via NTIS). This strain is more attenuated ($10^6$-fold in a mouse model; Buller et al: Decreased virulence of recombinant vaccinia virus expression vectors is associated with a thymidine kinase negative phenotype; Nature 317, 1985, 813-815) as compared to the usually used WR strain and is a safer vector for production purposes.

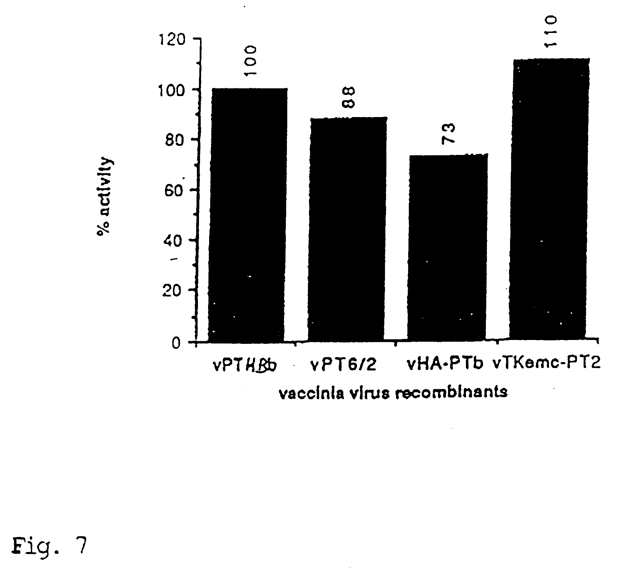

vPT6/2 was used in a similar way as described in Example I for the expression of prothrombin. The yields of biological activity of prothrombin in Vero cells can be derived from Fig. 7. For further conditions see legend Fig. 7.

Example III:

Plasmids for the insertion of prothrombin cDNA into the vaccinia virus hemagglutinin locus

In the prothrombin expressing recombinants as described in Examples I and II, the prothrombin cDNA was integrated into the viral thymidine kinase locus. Since inactivation of the viral tk-gene attenuates the virus, one may assume that this attenuation may also lead to a reduction in expression levels of foreign genes to be expressed.

Therefore, two viral recombinants were constructed which have the foreign gene integrated into the hemagglutinin locus of the vaccinia virus.

1. Construction of the vaccinia virus insertion plasmids pHAgpt-oFa and pHAgpt-oFb

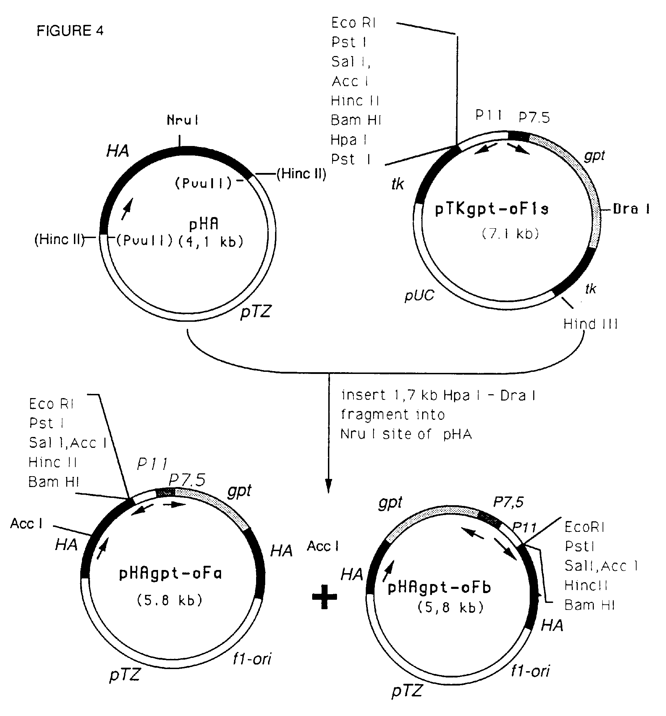

The basis vector, pHA, was constructed by ligating the 1.6 kb Hinc II fragment of the vaccinia hemagglutinin gene (Shida H.: Nucleotide sequence of the vaccinia virus hemagglutinin gene; Virology 150, 451-562) with the large Pvu II vector fragment of pTZ19R (Pharmacia, Inc.). The source of the HA-gene was the plasmid pVY5 (obtained from B. Moss). Into the single Nru I site of pHA the 1.7 kb Hpa I-Dra I gene cassette of plasmid pTKgpt-oF1s was inserted (Falkner, F.G., Moss B.: Escherichia coli gpt gene provides dominant selection for vaccinia virus open reading frame expression vectors; J. Virol. 1988; 62: 1849-1854). The cassette consists of the P11 promoter including a multiple cloning site and the gpt gene driven by the vaccinia virus P7.5 promoter. The two possible orientations were designated pHAgpt-oFa and pHAgpt-oFb. These plasmids are represented in Fig. 4.

2. Construction of the vaccinia virus insertion plasmids pHAgpt-PTa and pHAgpt-PTb

The plasmid pHAgpt-PTa was constructed by inserting the 2.0 kb EcoRI fragment of pTKgpt-PTHBb into EcoRI linearized and phosphatase treated plasmid pHAgpt-oFa.

Similarly, plasmid pHAgpt-PTb was constructed by inserting said 2.0 kb EcoRI fragment into the EcoRI linearized plasmid pHAgpt-oFb;

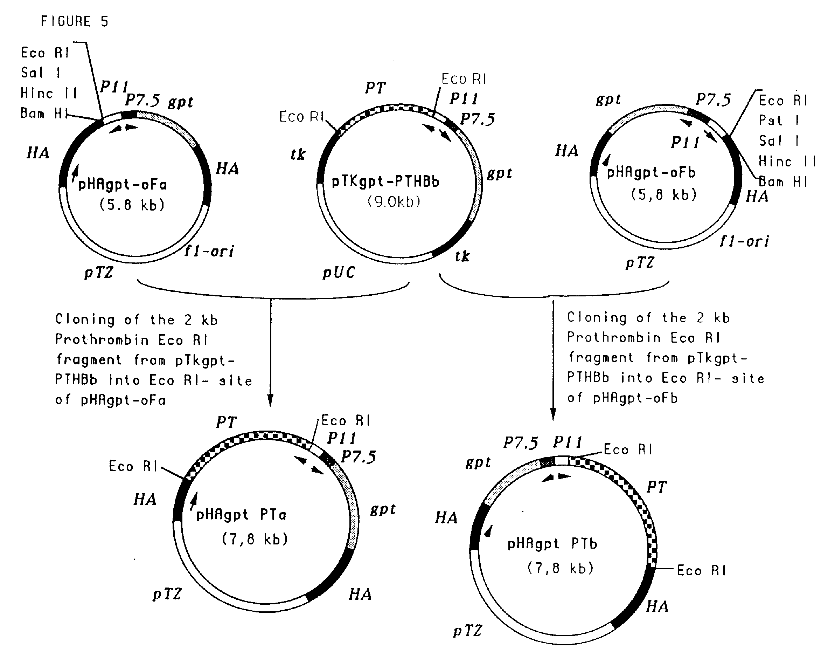

The above plasmids which are shown in Fig. 5 were used to obtain by in vivo recombination with vaccinia wild-type virus the recombinant viruses vHA-PTa and vHA-PTb.

Vero cells were infected with the above recombinant viruses in a similar way as described in Example II. The expression level of prothrombin in comparison to other viral recombinants can be derived from Fig. 7.

Example IV:

Construction of vaccinia virus recombinant vTKemc-PT2 and its use for the expression of prothrombin

In order to improve the levels of prothrombin expression a novel vaccinia virus recombinant was constructed, which is based on a very efficient hybrid vaccinia virus expression system. In this system, one virus expresses the bacteriophage T7 polymerase and a second virus expresses the target gene (prothrombin) under the control of the T7 promoter followed by the leader sequence of the encephalomyocarditis (EMC) virus (Elroy-Stein et al: Cap-independent translation of mRNA conferred by encephalomyocarditis virus 5' sequence improves the performance of the vaccinia virus/bacteriophage T7 hybrid expression system, Proc. Natl. Acad. Sci. (USA), 1989; 86, 6126-6130). This EMC leader confers cap-independent translation to the T7 transcripts and thus increases translational efficiency.

1. Construction of vaccinia virus insertion plasmid pTKemc-PT2

The 2.0 kb EcoRI fragment of the plasmid pTKgpt-PTHBb (Fig. 2), containing the prothrombin cDNA, was inserted into the single EcoRI site of the vector pTM3. Into the resulting plasmid pTKemc-PTx the prothrombin cDNA is inserted out-of-frame behind the initiation start codon provided by the vector pTM3. In order to correctly fuse the coding region with the start codon, an oligonucleotide directed mutagenesis was performed with pTKemc-PTx single.strand DNA and the oligonucleotide oPT9 (5'-AGC CTC GGA CGT GCG CCA TGG TAT TAT CGT-3'). Single stranded DNA was obtained from pTKemc-PTx by transfecting the plasmid into the E. coli strain NM 522 and superinfecting with helper phage M13 K07. In the resulting plasmid pTKemc-PT2, the correct primary structures of the fusion site around the initiation codon was confirmed by sequencing with the primer oPT2 ('5-CTG TGC ACA AGG CTA CAC-2').

By this measure the wild-type sequence of prothrombin is restored in the plasmid pTKemc-PT2.

The recombinant virus vTKemc-PT2 was then obtained by in vivo recombination techniques as described in Example I, followed by dominant selection of the recombinant virus.

**Claims**

**Claims for the following Contracting States : AT, BE, CH, LI, DE, DK, FR, GB, GR, IT, LU, NL, SE**

1. Insertion plasmid comprising a foreign cDNA sequence coding for a vitamin K-dependent blood factor or a functional equivalent thereof, at least one replicon, at least one selection marker, cloning sites and the vaccinia virus P11 promoter, said promoter and said foreign cDNA being flanked by non-essential DNA sequences of vaccinia virus, and the 3'-end of the promoter and the 5'-end of the downstream following foreign DNA sequence being in immediate proximity to one another by inserting the foreign DNA sequence in the correct open reading frame in that a restriction site is formed which corresponds to an identically engineered or natural restriction site at the 3'-end of the P11 promoter.

2. Plasmid according to claim 1, wherein the flanking DNA sequences of vaccinia virus correspond to the thymidine kinase gene.

3. Plasmid according to claim 1, wherein the flanking DNA sequences of vaccinia virus correspond to the hemagglutinin gene.

4. Plasmid according to claims 1 to 3, wherein the foreign cDNA codes for prothrombin.

5. Plasmid according to claim 4, comprising a novel EcoRI restriction endonuclease cleavage site by amending the prothrombin signal sequence in such a way that it comprises for proper insertion the DNA sequence coding for the two additional amino acids Asn and Ser at the 5'-end.

6. Plasmid according to claim 5, comprising the following partial promoter-foreign cDNA sequence:

   TATAA ATG AAT TCC GCGCAC--/   /--TAG-XXXTTC-60A-14C.

7. Plasmid according to claim 5, comprising the following partial promoter-foreign cDNA sequence:
   
   TATAA ATG AAT TCC GCGCAC--/   /--TAG--GAATTC--.

8. Plasmid according to one of the preceding claims, wherein a foreign cDNA sequence coding for vitamin K-dependent blood factor has been cloned into the multiple cloning site of plasmid pTKgpt-oFls in immediate proximity to the promoter.

9. Recombinant vaccinia virus obtainable by in vivo homologous recombination of wild-type vaccinia virus with a plasmid according to claims 1 to 8.

10. Recombinant vaccinia virus obtainable by in vivo homologous recombination of vaccinia virus strain #WR6/2 with the plasmids according to claims 1 to 8.

11. Vertebrate cell culture for the expression of vitamin K-dependent blood factor obtainable by infection of a host cell with recombinant vaccinia virus according to claims 9 and 10.

12. Cell culture according to claim 11, wherein the host cell is a mammalian liver or kidney cell.

13. Cell culture according to claim 12, wherein the host cells are Vero, CV1, BSC1, BHK or RK13 cells.

14. Process for the recombinant production of a vitamin K-dependent blood factor comprising infection of mammalian host cells with a recombinant vaccinia virus according to claims 9 and 10, culturing said cells, expressing said vitamin K-dependent blood factor and recovering the same.

15. Process according to claim 14, wherein said host cells are Vero, CV1, BSC1, BHK or RK13 cells.

16. Process according to claim 15, wherein host cells RK13 are used and the foreign gene lacks the signal sequence.

17. Process according to claim 14, wherein prior to infection the cells have reached confluency.

18. Process according to claim 14, wherein the culturing of cells and/or expression of said vitamin K-dependent blood factor is carried out in the presence of vitamin K.

19. Process according to claim 14, wherein culturing of said infected cells is carried out in a high density system.

20. Process according to claim 14, wherein culturing of said infected cells is carried out in a micro-carrier culture or a hollow-fiber system.

21. Process according to claim 14, wherein prothrombin is prepared.

22. Process according to claim 14, wherein the recovering of the said vitamin K-dependent blood factor is carried out for 48 to 96 h, preferably 60 to 72 h, after infection.

**Claims for the following Contracting State : ES**

1. A process for the preparation of an insertion plasmid comprising a foreign cDNA sequence coding for a vitamin K-dependent blood factor or a functional equivalent thereof, characterized by constructing a plasmid which comprises at least one replicon, at least one selection marker, cloning sites and the vaccinia virus P11 promoter, said promoter and said foreign cDNA being flanked by non-essential DNA sequences of vaccinia virus, and in that the 3'-end of the promoter and the 5'-end of the downstream following foreign DNA sequence being brought in immediate proximity to one another by inserting the foreign DNA sequence in the correct open reading frame in that a restriction site is formed which corresponds to an identical engineered or natural restriction site at the 3'-end of the P11 Ppomoter.

2. The process according to claim 1, wherein the flanking DNA sequences of vaccinia virus correspond to the thymidine kinase gene.

3. The process according to claim 1, wherein the flanking DNA sequences of vaccinia virus correspond to the hemagglutinin gene.

4. The process according to claims 1 to 3, wherein the foreign cDNA codes for prothrombin.

5. The process according to claim 4, characterized in that the plasmid is constructed in a way that a novel EcoRI restriction endonuclease cleavage site is formed by amending the prothrombin signal sequence in such a way that it comprises for proper insertion the DNA sequence coding for the two additional amino acids Asn and Ser at the 5'-end.

6. The process according to claim 5, characterized in that the plasmid comprises the following partial promoter-foreign cDNA sequence:

```
TATAA ATG AAT TCC GCGCAC--/   /--TAG-XXXTTC-60A-14C.
```

7. The process according to claim 5, characterized in that the plasmid comprises the following partial promoter-foreign cDNA sequence:

```
TATAA ATG AAT TCC GCGCAC--/   /--TAG--GAATTC--.
```

8. The process according to one of the preceding claims, wherein a foreign cDNA sequence coding for vitamin K-dependent blood factor has been cloned into the multiple cloning site of plasmid pTKgpt-oFIs in immediate proximity to the promoter.

9. A process for the preparation of recombinant vaccinia virus, characterized by in vivo homologous recombination of wild-type vaccinia virus with a plasmid according to claims 1 to 8.

10. A process for the preparation of recombinant vaccinia virus, characterized by in vivo homologous recombination of vaccinia virus strain #WR6/2 with the plasmids according to claims 1 to 8.

11. A process for the preparation of a vertebrate cell culture for the expression of vitamin K-dependent blood factor, characterized by infection of a host cell with recombinant vaccinia virus according to claims 9 and 10.

12. The process according to claim 11, wherein the host cell is a mammalian liver or kidney cell.

13. The process according to claim 12, wherein the host cells are Vero, CV1, BSC1, BHK or RK13 cells.


**Patentansprüche**

**Patentansprüche für folgende Vertragsstaaten : AT, BE, CH, DE, DK, FR, GB, GR, IT, LI, LU, NL, SE**

1. Insertionsplasmid, umfassend eine fremde cDNA-Sequenz, die für einen von Vitamin K abhängigen Blutfaktor oder ein funktionelles Äquivalent davon codiert, mindestens ein Replikon, mindestens einen Selektionsmarker, Klonierungsstellen und den P11-Promotor des Kuhpockenvirus, wobei der Promotor und die fremde cDNA von nicht-essentiellen DNA-Sequenzen des Kuhpockenvirus flankiert werden und das 3'-Ende des Promotors und das 5'-Ende der stromabwärts folgenden fremden DNA-Sequenz sich in unmittelbarer Nähe voneinander befinden, und zwar durch Insertion der fremden DNA-Sequenz in den korrekten offenen Leserahmen, indem eine Restriktionsstelle gebildet wird, die einer auf identische Weise erzeugten oder natürlich auftretenden Restriktionsstelle am 3'-Ende des P11-Promotors entspricht.

2. Plasmid nach Anspruch 1, wobei die flankierenden DNA-Sequenzen des Kuhpockenvirus dem Thymidinkinasegen entsprechen.

3. Plasmid nach Anspruch 1, wobei die flankierenden DNA-Sequenzen des Kuhpockenvirus dem Hämagglutiningen entsprechen.

4. Plasmid nach einem der Ansprüche 1 bis 3, wobei die fremde cDNA für Prothrombin codiert.

5. Plasmid nach Anspruch 4, umfassend eine neue EcoRI-Restriktionsendonuclease-Schnittstelle, indem die Prothrombinsignalsequenz in einer solchen Weise geändert wird, daß sie für die korrekte Insertion die DNA-Sequenz umfaßt, die für die beiden zusätzlichen Aminosäuren Asn und Ser am 5'-Ende codiert.

6. Plasmid nach Anspruch 5, umfassend die folgende Promotor-Fremd-cDNA-Teilsequenz:

```
TATAA ATG AAT TCC GCGCAC--/   /--TAG-XXXTTC-60A-14C.
```

7. Plasmid nach Anspruch 5, umfassend die folgende Promotor-Fremd-cDNA-Teilsequenz:

```
TATAA ATG AAT TCC GCGCAC--/   /--TAG--GAATTC--.
```

8. Plasmid nach einem der vorstehenden Ansprüche, wobei die fremde cDNA-Sequenz, die für einen von Vitamin K abhängigen Blutfaktor codiert, in die mehrfache Klonierungsstelle des Plasmids pTKgpt-oFls in unmittelbarer Nähe des Promotors kloniert ist.

9. Rekombinantes Kuhpockenvirus, erhältlich durch homologe in vivo-Rekombination des Wildtyp-Kuhpockenvirus mit einem Plasmid nach einem der Ansprüche 1 bis 8.

10. Rekombinantes Kuhpockenvirus, erhältlich durch homologe in vivo-Rekombination des Kuhpockenvirusstamms WR6/2 mit den Plasmiden nach einem der Ansprüche 1 bis 8.

11. Vertebratenzellkultur zur Expression eines von Vitamin K abhängigen Blutfaktors, erhältlich durch Infektion einer Wirtszelle mit einem rekombinanten Kuhpockenvirus nach Anspruch 9 oder 10.

12. Zellkultur nach Anspruch 11, wobei es sich bei der Wirtszelle um eine Säugerleberzelle oder Säugernierenzelle handelt.

13. Zellkultur nach Anspruch 12, wobei es sich bei den Wirtszellen um Vero-, CV1-, BSC1-, BHK- oder RK13-Zellen handelt.

14. Verfahren zur rekombinanten Herstellung eines von Vitamin K abhängigen Blutfaktors, umfassend die Infektion von Säugerwirtszellen mit einem rekombinanten Kuhpockenvirus nach einem der Ansprüche 9 und 10, das Züchten der Zellen, die Expression des von Vitamin K abhängigen Blutfaktors und die Gewinnung des Faktors.

15. Verfahren nach Anspruch 14, wobei es sich bei den Wirtszellen um Vero-, CV1-, BSC1-, BHK- oder RK13-Zellen handelt.

16. Verfahren nach Anspruch 15, wobei RK13-Wirtszellen verwendet werden und den fremden Genen die Signalsequenz fehlt.

17. Verfahren nach Anspruch 14, wobei die Zellen vor der Infektion die Konfluenz erreicht haben.

18. Verfahren nach Anspruch 14, wobei das Züchten der Zellen und/oder die Expression des von Vitamin K abhängigen Blutfaktors in Gegenwart von Vitamin K durchgeführt werden.

19. Verfahren nach Anspruch 14, wobei das Züchten der infizierten Zellen in einem System mit hoher Dichte durchgeführt wird.

20. Verfahren nach Anspruch 14, wobei das Züchten der infizierten Zellen in einer Mikroträgerkultur oder einem Hohlfasersystem durchgeführt wird.

21. Verfahren nach Anspruch 14, wobei Prothrombin hergestellt wird.

22. Verfahren nach Anspruch 14, wobei die Gewinnung des von Vitamin K abhängigen Blutfaktors 48 bis 96 Stunden und vorzugsweise 60 bis 72 Stunden nach der Infektion durchgeführt wird.

**Patentansprüche für folgenden Vertragsstaat : ES**

1. Verfahren zur Herstellung eines Insertionsplasmids, umfassend eine fremde cDNA-Sequenz, die für einen von Vitamin K abhängigen Blutfaktor oder ein funktionelles Äquivalent davon codiert, gekennzeichnet durch Konstruktion eines Plasmids, das mindestens ein Replikon, mindestens einen Selektionsmarker, Klonierungsstellen und den P11-Promotor des Kuhpockenvirus umfaßt, wobei der Promotor und die fremde cDNA von nicht-essentiellen DNA-Sequenzen des Kuhpockenvirus flankiert werden, und dadurch, daß das 3'-Ende des Promotors und das 5'-Ende der stromab- wärts folgenden fremden DNA-Sequenz in unmittelbarer Nähe zueinander gebracht werden, und zwar durch Insertion der fremden DNA-Sequenz in den korrekten offenen Leserahmen, indem eine Restriktionsstelle gebildet wird, die einer erzeugten oder natürlich auftretenden identischen Restriktionsstelle am 3'-Ende des P11-Promotors entspricht.

2. Verfahren nach Anspruch 1, wobei die flankierenden DNA-Sequenzen des Kuhpockenvirus dem Thymidinkinasegen entsprechen.

3. Verfahren nach Anspruch 1, wobei die flankierenden DNA-Sequenzen des Kuhpockenvirus dem Hämagglutiningen entsprechen.

4. Verfahren nach einem der Ansprüche 1 bis 3, wobei die fremde cDNA für Prothrombin codiert.

5. Verfahren nach Anspruch 4, dadurch gekennzeichnet, daß das Plasmid auf eine solche Weise konstruiert wird, daß eine neue EcoRI-Restriktionsendonuclease-Schnittstelle gebildet wird, indem die Prothrombinsignalsequenz in einer solchen Weise geändert wird, daß sie für die korrekte Insertion die DNA-Sequenz umfaßt, die für die beiden zusätzlichen Aminosäuren Asn und Ser am 5'-Ende codiert.

6. Verfahren nach Anspruch 5, umfassend die folgende Promotor- Fremd-cDNA-Teilsequenz:

```
TATAA ATG AAT TCC GCGCAC--/  /--TAG-XXXTTC-60A-14C.
```

7. Verfahren nach Anspruch 5, umfassend die folgende Promotor- Fremd-cDNA-Teilsequenz:

```
TATAA ATG AAT TCC GCGCAC--/   /--TAG--GAATTC--.
```

8. Verfahren nach einem der vorstehenden Ansprüche, wobei die fremde cDNA-Sequenz, die für einen von Vitamin K abhängigen Blutfaktor codiert, in die mehrfache Klonierungsstelle des Plasmids pTKgpt-oFls in unmittelbarer Nähe des Promotors kloniert ist.

9. Verfahren zur Herstellung eines rekombinanten Kuhpockenvirus, gekennzeichnet durch homologe in vivo-Rekombination des Wildtyp-Kuhpockenvirus mit einem Plasmid nach einem der Ansprüche 1 bis 8.

10. Verfahren zur Herstellung eines rekombinanten Kuhpockenvirus, gekennzeichnet durch homologe in vivo-Rekombination des Kuhpockenvirus- stamms WR6/2 mit den Plasmiden nach einem der Ansprüche 1 bis 8.

11. Verfahren zur Herstellung einer Vertebratenzellkultur zur Expression eines von Vitamin K abhängigen Blutfaktors, gekennzeichnet durch Infektion einer Wirtszelle mit einem rekombinanten Kuhpockenvirus nach Anspruch 9 oder 10.

12. Verfahren nach Anspruch 11, wobei es sich bei der Wirtszelle um eine Säugerleberzelle oder Säugernierenzelle handelt.

13. Verfahren nach Anspruch 12, wobei es sich bei den Wirtszellen um Vero-, CV1-, BSC1-, BHK- oder RK13-Zellen handelt.

## Revendications

**Revendications pour les Etats contractants suivants : AT, BE, CH, DE, DK, FR, GB, GR, IT, LI, LU, NL, SE**

1. Plasmide d'insertion comprenant une séquence d'ADNc étranger codant pour un facteur sanguin dépendant de la vitamine K ou un de ses équivalents fonctionnels, au moins un réplicon, au moins un marqueur de sélection, des sites de clonage et le promoteur P11 du virus de la vaccine, ce promoteur et cet ADNc étranger étant flanqués par des séquences d'ADN non essentielles du virus de la vaccine, et l'extrémité 3' du promoteur et l'extrémité 5' de la séquence d'ADN étranger suivant en aval étant à proximité immédiate l'une de l'autre par insertion de la séquence d'ADN étranger dans le cadre de lecture ouvert correct en ce sens qu'un site de restriction est formé, qui correspond à un site de restriction fabriqué de manière identique ou naturel à l'extrémité 3' du promoteur P11.

2. Plasmide selon la revendication 1, dans lequel les séquences d'ADN adjacentes du virus de la vaccine correspondent au gène de la thymidine kinase.

3. Plasmide selon la revendication 1, dans lequel les séquences d'ADN adjacentes du virus de la vaccine correspondent au gène de l'hémagglutinine.

4. Plasmide selon les revendications 1 à 3, dans lequel l'ADNc étranger code pour la prothrombine.

5. Plasmide selon la revendication 4, contenant un nouveau site de coupure par l'endonucléase de restriction EcoRI en modifiant la séquence signal de la prothrombine de telle manière qu'elle contienne pour une insertion appropriée la séquence d'ADN codant pour les deux amino-acides supplémentaires Asn et Ser à l'extrémité 5'.

6. Plasmide selon la revendication 5, comprenant la séquence promoteur partiel-ADNc étranger suivante :

```
TATAA ATG AAT TCC GCGCAC--/ /--TAG-XXXTTC-6OA-14C.
```

7. Plasmide selon la revendication 5, comprenant la séquence promoteur partiel-ADNc étranger suivante :

```
TATAA ATG AAT TCC GCGCAC--/ /--TAG--GAATTC--.
```

8. Plasmide selon l'une quelconque des revendications précédentes, dans lequel une séquence d'ADNc étranger codant pour un facteur sanguin dépendant de la vitamine K a été clonée dans le site de clonage multiple du plasmide pTKgpt-oF1s à proximité immédiate du promoteur.

9. Virus de la vaccine recombinant pouvant être obtenu par recombinaison homologue in vivo d'un virus de la vaccine du type sauvage avec un plasmide selon les revendications 1 à 8.

10. Virus de la vaccine recombinant pouvant être obtenu par recombinaison homologue in vivo de la souche de virus de la vaccine n° WR6/2 avec les plasmides selon les revendications 1 à 8.

11. Culture cellulaire de vertébré pour l'expression du facteur sanguin dépendant de la vitamine K pouvant être obtenue par l'infection d'une cellule hôte avec un virus de la vaccine recombinant selon les revendications 9 à 10.

12. Culture cellulaire selon la revendication 11, dans laquelle la cellule hôte est une cellule de foie ou de rein de mammifère.

13. Culture cellulaire selon la revendication 12, dans laquelle les cellules hôtes sont des cellules Vero, CV1, BSC1, BHK ou RK13.

14. Procédé pour la production par recombinaison d'un facteur sanguin dépendant de la vitamine K comprenant l'infection de cellules hôtes de mammifère avec un virus de la vaccine recombinant selon les revendications 9 et 10, la culture de ces cellules, l'expression de ce facteur sanguin dépendant de la vitamine K et la récupération de celui-ci.

15. Procédé selon la revendication 14, dans lequel ces cellules hôtes sont des cellules Vero, CV1, BSC1, BHK ou RK13.

16. Procédé selon la revendication 15, dans lequel des cellules hôtes RK13 sont utilisées et le gène étranger est dépourvu de la séquence signal.

17. Procédé selon la revendication 14, dans lequel, avant l'infection, les cellules ont atteint la confluence.

18. Procédé selon la revendication 14, dans lequel la culture des cellules et/ou l'expression de ce facteur sanguin dépendant de la vitamine K est effectuée en présence de vitamine K.

19. Procédé selon la revendication 14, dans lequel la culture de ces cellules infectées est effectuée dans un système à haute densité.

20. Procédé selon la revendication 14, dans lequel la culture de ces cellules infectées est effectuée dans une culture sur micro-support ou dans un système à fibres creuses.

21. Procédé selon la revendication 14, dans lequel on prépare la prothrombine.

22. Procédé selon la revendication 14, dans lequel la récupération de ce facteur sanguin dépendant de la vitamine K est effectuée pendant 48 à 96 heures, de préférence pendant 60 à 72 heures, après l'infection.

**Revendications pour l'Etat contractant suivant : ES**

1. Procédé de préparation d'un plasmide d'insertion comprenant une séquence d'ADNc étranger codant pour un facteur sanguin dépendant de la vitamine K ou un équivalent fonctionnel de celui-ci, caractérisé en ce qu'on construit un plasmide qui comprend au moins un réplicon, au moins un marqueur de sélection, des sites de clonage et le promoteur P11 du virus de la vaccine, ce promoteur et cet ADNc étranger étant flanqués par des séquences d'ADN non essentielles du virus de la vaccine, et en ce que l'extrémité 3' du promoteur et l'extrémité 5' de la séquence d'ADN étranger suivant en aval sont amenées à proximité immédiate l'un de l'autre dans le cadre de lecture ouvert correct en ce sens qu'un site de restriction est formé, qui correspond à un site de restriction identique fabriqué ou naturel à l'extrémité 3' du promoteur P11.

2. Procédé selon la revendication 1, dans lequel les séquences d'ADN adjacentes du virus de la vaccine correspondent au gène de la thymidine kinase.

3. Procédé selon la revendication 1, dans lequel les séquences d'ADN adjacentes du virus de la vaccine correspondent au gène de l'hémagglutinine.

4. Procédé selon les revendications 1 à 3, dans lequel l'ADNc étranger code pour la prothrombine.

5. Procédé selon la revendication 4, caractérisé en ce que le plasmide est construit de telle manière qu'un nouveau site de coupure par l'endonucléase de restriction EcoRI est formé en modifiant la séquence signal de la prothrombine de telle manière qu'elle contienne pour une insertion appropriée la séquence d'ADN codant pour les deux amino-acides supplémentaires Asn et Ser à l'extrémité 5'.

6. Procédé selon la revendication 5, caractérisé en ce que le plasmide comprend la séquence promoteur partiel-ADNc étranger suivante :

```
TATAA ATG AAT TCC GCGCAC--/ /--TAG-XXXTTC-6OA-14C.
```

7. Procédé selon la revendication 5, caractérisé en ce que le plasmide comprend la séquence promoteur partiel-ADNc étranger suivante :

```
TATAA ATG AAT TCC GCGCAC--/ /--TAG--GAATTC--.
```

8. Procédé selon l'une quelconque des revendications précédentes, dans lequel une séquence d'ADNc codant pour le facteur sanguin dépendant de la vitamine K a été clonée dans le site de clonage multiple du plasmide pTKgpt-oF1s à proximité immédiate du promoteur.

9. Procédé pour la préparation d'un virus de la vaccine recombinant, caractérisé par une recombinaison homologue in vivo du virus de la vaccine du type sauvage avec un plasmide selon les revendications 1 à 8.

10. Procédé pour la préparation d'un virus de la vaccine recombinant, caractérisé par une recombinaison homologue in vivo de la souche de virus de la vaccine n° WR6/2 avec les plasmides selon les revendications 1 à 8.

11. Procédé pour la préparation d'une culture cellulaire de vertébré pour l'expression d'un facteur sanguin dépendant de la vitamine K, caractérisé par l'infection d'une cellule hôte avec un virus de la vaccine recombinant selon les revendications 9 et 10.

12. Procédé selon la revendication 11, dans lequel la cellule hôte est une cellule de foie ou de rein de mammifère.

13. Procédé selon la revendication 12, dans lequel les cellules hôtes sont des cellules Vero, CV1, BSC1, BHK ou RK13.

FIGURE 1

vPTHBa

Pst I    *PTHB*    Pst I

1.6    0.4

*tet* r    pIIH13
(5.8kbp)

Pst I    Pst I

*pKT218*

-insert 1.6kb Pst I PTHB
fragment into M13mp18
-isolate ss-DNA

*PTHB*    Pst I

Pst I    1.6

mp18.PTHB.1    ssDNA

-oligonucleotide
directed mutagenesis
-screening/sequencing

Eco RI
Pst I
Sal I, Acc I
Hinc II
Bam HI
Hpa I
Pst I

*PTHB*    Pst I

Eco RI
Pst I    1.6

mp18.PTHB.2
(8.6kb)

*M13.mp18*

PstI    *PTHB*    EcoRI

Pst I    Pi1    P7.5

*tk*    pTKgpt-PTHBa
(9.1kb)    *gpt*

*tk*

*pUC*

-insert 0.4kb Pst I
PTBH fragment

*PTHB*    EcoRI

Pst I    P11
P7.5

*tk*    pTKgpt-PTHBi
(8.7kb)    *gpt*

*tk*

*pUC*

-insert modified
1.6kb fragment into
pTKgpt-oF1s

*tk*    P11    P7.5

pTKgpt-oF1s    *gpt*
(7.1 kb)

*tk*

*pUC*    Hind III

FIGURE 2

EP 0 512 011 B1

# FIGURE 3

| vaccinia late promotor consensus | $\begin{smallmatrix}AA\\TT\end{smallmatrix}$ TAA AT GPuPu | | | | |
|---|---|---|---|---|---|
| | | *met* | *asn ser* | | |
| P11 WT | | TATAA ATG | AAT TCC--/ /--TAG-- | | |
| | | *met* | *ala his* | | |
| prothrombin cDNA | -16G-XXX | ATG | GCG CAC --------/ /---TAG-XXXTTC-(60)A-(14)C- | | |
| | | *met* | *asn ser ala his* | | |
| vPTHB a | | TATAA ATG | AAT TCC GCG CAC--/ /--TAG--XXXTTC-(60)A-(14)C- | | |
| | | *met* | *asn ser ala his* | | |
| vPTHB b | | TATAA ATG | AAT TCC GCG CAC--/ /--TAG--GAATTC-- | | |
| | | *Eco RI* | | | *Eco RI* |

FIGURE 4

insert 1,7 kb Hpa I – Dra I
fragment into
Nru I site of pHA

FIGURE 5

Eco RI
Sal I
Hinc II
Bam HI

P11
P7.5 gpt

HA

pHAgpt-oFa
(5.8 kb)

HA

f1-ori

pTZ

PT

Eco RI
Eco RI

P11
P7.5

tk

pTKgpt-PTHBb
(9.0kb)

gpt

pUC

tk

gpt

P7.5

P11

Eco RI
Pst I
Sal I
Hinc II
Bam HI

HA

pHAgpt-oFb
(5,8 kb)

HA

f1-ori

pTZ

Cloning of the 2 kb
Prothrombin Eco RI
fragment from pTkgpt-
PTHBb into Eco RI- site
of pHAgpt-oFa

Cloning of the 2 kb
Prothrombin Eco RI
fragment from pTkgpt-
PTHBb into Eco RI- site
of pHAgpt-oFb

PT

Eco RI
P11
P7.5

Eco RI

HA

pHAgpt PTa
(7,8 kb)

gpt

pTZ

HA

P7.5 P11

gpt

Eco RI

PT

HA

pHAgpt PTb
(7,8 kb)

Eco RI

HA

pTZ

EP 0 512 011 B1

Fig. 6

Construction scheme of the vaccinia virus insertion plasmid pTKemc-PT2

% activity

120 — 100
100 — 88
80 — 73
60
40 — 110
20
0

vPTHβb    vPT6/2    vHA-PTb    vTKemc-PT2

vaccinia virus recombinants

Fig. 7